# EUROPEAN PATENT APPLICATION

(11) **EP 1 258 229 A1**
(43) Date of publication of application: **20.11.2002**
(21) Application number: 01111760.3
(22) Date of filing: 15.05.2001
(51) Int. Cl.: A61F 2/06

(54) **Flexible and elastic vascular stents and grafts**

(71) Applicant: VASCULAR TECHNOLOGIES, INC., New Hope, PA 18938 (US)
(72) Inventor: Wakhloo, Ajay K., Key Biscayne, FL 33149 (US); Kalidindi, Surya R., Tallahassee, FL 32308 (US); Gobran, Riad H., New Hope, PA 18938 (US); Khan, Yusuf, S., Philadelphia, PA 19106 (US); Naidu, Naveen, Philadelphia, PA 19104 (US); Ko, Frank K., Philadelphia, PA 19124 (US)
(74) Representative: Harrison, Michael Charles

(57) **Abstract**

A device for insertion into a body as a stent or graft to a predetermined position in a body location comprising a braided stent made of heat-settable polymer. The stent is compressed axially to a shorter length and/or molded to a desired shape, and heat-set in a predetermined configuration The device may also include non heat-settable filaments to enhance its performance as for example metal fibers to impart radio opacity. The stent is adapted to be axially extended for insertion into a delivery systems used to introduce the device to a specific body location, whereby it returns to substantially its heat-set predetermined configuration. The device may also include a debris protection component including a distal end and a proximal end, the component being attached to the distal end of the stent device preferably used with an insertion guide wire with the distal end fixedly mounted to the guide wire for closure of the distal end upon removal of the debris protection component to entrap body debris at the body location. The debris protection component may be fixedly or removeably attached to the stent device. In one embodiment, the stent includes a natural or synthetic coating on at least one surface of the stent.

## Description

### FIELD OF THE INVENTION

This invention relates to medical devices. More particularly, the invention relates to flexible and elastic devices produced using heat-settable polymer filaments that can be used as stents, grafts and distal protection systems.

### BACKGROUND OF THE INVENTION

A wide variety of medical procedures require the insertion of tubular prostheses into the body of a patient A tubular prosthesis can be implanted within an already existing body lumen, such as in a blood vessel, the bilary tract, or the esophagus. As an intravascular graft, it serves for treating aneurysms in cerebral or coronary arteries, stenosis in native arteries or in saphenous-vein grafts. A tubular prosthesis can also be used as a synthetic graft, for example, in performing a by-pass of a blocked blood vessel or vessels that have been infiltrated by tumor tissue. Tubular prostheses can also be inserted into other areas of the body such as urinary tracts or respiratory tracts for several purposes, one of which is to support the inner wall of the vessel or tract. Prostheses of these types are generally referred to as either stents or grafts. Grafts are generally used to replace a portion of a bodily lumen, whereas a stent is inserted into a lumen and left there for the purpose of providing support against the collapse of the lumen.

The devices discussed above often require a combination of properties that are difficult to mutually possess. These medical devices must be strong, flexible, elastic, biocompatible, sometimes porous, radio-opaque, and sized correctly. Vascular stents used in treating aneurysms in blood vessels, must be flexible enough to bend and conform to the shape of the blood vessels into which they are inserted. Blood vessels are not straight and rigid tubes. Blood vessels are generally elastic and their inner diameter fluctuates as the conditions of the body change, yet blood vessels are also strong enough to maintain sufficient blood pressure to pump blood throughout the body.

### STENTS

A stent used to treat an aneurysm in a blood vessel must be flexible enough and strong enough to support the damaged vessel. Most of the current vascular stents are radially expandable, such that upon expansion they become lodged in place in the blood vessel. Radial expansion can be accomplished in a number of ways. One method of providing radial expansion to a vascular stent is to use materials with some kind of memory function. This is currently achieved by use of certain metal alloys that show a "shape-memory" effect, i.e., upon heating return to some predetermined shape. Another more common approach is to construct the stent using metal fibers. In order to provide a tubular structure having the necessary radial strength to support a blood vessel, it is often necessary to use relatively large diameter metal fibers.

Most of the stents currently used in treating aneurysms and stenosis are made entirely of metal, generally delivered on a balloon and expanded to the desired diameter (hence the term balloon-expanded wire stent). The disadvantage of most current stents, which are made entirely of metal, is that they are quite stiff, making deployment within a blood vessel extremely difficult, especially in a bent location. Stents made entirely of metal are also prone to other disadvantages. These stents can be too stiff radially, thereby causing complications after insertion. Stents made of less rigid materials than metal may lack the required radial stiffness and suffer from collapse themselves, as there is no inherent mechanism keeping the stents open. Stents can be of any length and subsequently cut by the inserting physician to the exact length necessary. Stents cut to fit typically fray or unravel. The exposed frayed tips of the fibers can be sharp in comparison to the walls of a blood vessel and could cause damage. Additionally, the exposed tips of the fibers can be difficult to handle and can lead to complications during insertion into blood vessels.

The cerebrovascular system is unique as compared with other areas of the body since it represents a low-impedance system. Cerebral vessels run into a watery medium called cerebrospinal fluid. They are tortuous and fragile. Their walls are differently structured, as they lack continuity of their muscular layers, as well as internal elastic fibers. Thus, incidence of aneurysm formation is much higher than elsewhere in the body. Stents or stent-like grafts have been proposed for cerebral aneurysm treatment Most of the stents currently in use are too stiff to be navigated into the cerebrovascular system. Furthermore, tiny branches extend from the large aneurysm-harboring vessels called perforators, which supply blood to normal brain tissue. Occlusion of these vessels generally leads to a devastating infarction (stroke). Currently available self-expanding stents (such as the Wallstent) have the disadvantage of 1) the filament diameter is too large, increasing the risk of occluding friable perforating vessels and 2) Wallstent flares at both ends, thus carrying the risk of closing the origin of perforation by structural damage (crossover tapering to the vessel wall) with possible clot formation.

### GRAFTS

In by-pass surgery, a patient's blocked or clogged artery or vein can be circumvented by using a piece of his or her own saphenous vein. However, often such a natural graft is not available, or the patient would not be able to withstand the additional surgery. In those instances synthetic grafts are often used. Synthetic vascular grafts require many properties in order to be effective. The grafts need to be flexible and elastic, yet strong enough to maintain the blood pressure of the patient without rupture or excessive expansion. Synthetic graft materials are often made from the same materials used in stents. Synthetic grafts are often difficult to produce in that they must be matched to the native artery with respect to the size and geometry of the native artery, as well as the physiological and biological properties of the native artery.

### PRIOR ART DEFICIENCIES

Some of the prior art discloses stents of some practical use. For example, Palmaz U.S. Patent No. 4,739,762 discloses a thin-walled tube of metal or plastic, which is expandable radially. Ryan et al U.S. Patent No. 5,108,416 also discloses both rolled plastic and metal stents.

Beck et al U.S. Patent No. 5,147,385 discloses that heat treatment may be used to soften the stent when it is in the body, at temperatures of 45-75 °C. In contrast, Hull U.S. Patent No. 5,192,297 discloses a self-expanding stent which is radially expanded. This patent is typical of the expandable stent designs, wherein the expansion takes place because force is applied to the stent in a radial direction. Solar U.S. Patent No. 5,403,341 teaches a wire stent and is not really a relevant reference.

Shors U.S. Patent No. 4,969,896 teaches a tube or stent with longitudinal ribs. Wallsten U.S. Patent No. 4,954,126 teaches a prosthesis comprising a flexible tubular body which has a diameter that is variable by axial movement of the ends. It includes rigid but flexible thread elements in a helix configuration. Wallsten teaches that the preferred stent has a self-expanding property, obtained by including strings or bands extending parallel and axially with the surface of the body. Pinchuk et al U.S. Patent No. 5,163,951 teaches a mesh composite made from a durable material and a second material having a melting point less than the first material, so that heating the graft bonds the components to form a porous, compliant graft with outer reinforcement.

Clouse U.S. Patent No. 5,211,658 discloses a two component system in which the inner part is expanded. Brancato U.S. Patent No. 5,458,636 uses longitudinal threads and lateral fibers used with a bioabsorbale sheet. Thompson U.S. Patent No. 5,718,159 and his related U.S. Patent No. 5,758,562 disclose a braided structure of metal or polymer with polymeric multi-filiment yarns which are heat-set prior to formation of the final device.

Thus there is still a need in the art for vascular prostheses, including stents and grafts, which are strong, flexible in both the radial and longitudinal directions, and elastic.

It would be of great advantage in the art if such a vascular prosthesis could be developed.

Another advantage is to provide a device which can be inserted without embolic debris, such as clots, dislodged plaques and such from the vessel wall being discharged up-stream in the blood flow.

Yet another object of the present invention is to provide simple, effective stent, graft and distal protection systems with multiple uses in virtually all medically related procedures using such devices.

Other objects will appear hereinafter.

### SUMMARY OF THE INVENTION

It has now been discovered that the above and other objects of the present invention may be accomplished in the following manner. The unique aspect of this invention is the construction of a tubular structure, produced by a sequence of steps involving one or more of the following, and used as a prosthesis such as a vascular stent, vascular graft or distal protection device:
1. braiding of (mono-or multi-) filaments of heat-settable polymers (Type 1) or co-braiding said filaments with non heat-settable filaments (metal, non-metal or both) (Type 2 structure),
2. heat-setting the tubular device to a desired shape after compressing it axially or molding it by convenient means,
3. coating the tubular structure either partially or fully to mitigate fraying of the ends and to improve anchorage, radio opaqueness or to facilitate local delivery of drugs, hormones or growth factors,
4. fusion of the ends by trimming and/or heat treatment also to prevent fraying.

Another aspect of the invention is its deployment, which is accomplished by launching the stent into the inner lumen of a delivery catheter and pushing it out at the location of choice using a balloon catheter or other suitable means.

The tubular structures are to be designed to have the desired geometry (diameter and length) depending on the size of the vascular vessel involved. An inherent property of these structures is that they can be stretched to a much smaller diameter for ease of insertion into a delivery catheter, but regain their original diameter after deployment.

There are at least two preferred embodiments of the invention:
1- The TYPE 1 structure is composed of heat-settable polymeric fibers. The fibers used can be mono-filament or multi-filament or a combination of both in order to allow for varying degrees of coverage.
2- The TYPE 2 structure is composed of at least one heat-settable polymeric fiber co-braided with either elastomeric or non-elastomeric fibers. The fibers used can be mono-filament or multi-filament or a combination of both in order to allow for varying degrees of coverage.

After fabrication of TYPE 1 or TYPE 2 structures, they are compressed axially or forced to a specific shape and then heat-set in that configuration, inducing memory. This structure then can be stretched to a smaller diameter for deployment and is capable of recovering to the heat-set configuration immediately after deployment.

Both types of structures described above can be given a coating on each end of the tubular prosthesis to prevent fraying and to help anchor the ends. This can also be accomplished by application of heat to the ends to fuse the protruding fibers together. A coating can also be provided on one or both sides of the stent to permit treatment or to cause the coating itself to swell upon contact with body fluids. This may be desirable when the stent is used for the treatment of aneurysms to minimize seepage of blood to the aneurysm itself.

Additionally, another aspect of the invention is the incorporation of a number of metallic fibers (TYPE 2 structure) by co-braiding with the polymeric fibers to provide easy detection by X-rays, i.e., to render the systems radio opaque. Radio Opacity for X-ray detection can be imparted to the stents, grafts and distal protection systems by coating the structures using various techniques"
a) Dip coating: radio opaque powder such as tantalum is thoroughly mixed with a biocompatible adhesive to form a suspension. The ends of the braided structures or the entire structures are dipped into the suspension and the resulting coating is allowed to dry in air and then cured in an oven at 160°F for five to ten minutes.
b) Vapor deposition: the structure to be coated and a radio opaque powder such as titanium are introduced in a vapor deposition chamber. The chamber is evacuated and then filled with an inert gas to prevent oxidation of the metaL The filaments in the chamber are then heated to a temperature high enough to vaporize the metal and deposit on the structure to be coated. Several such coatings may be required to achieve uniformity.
c) Gold sputtering: the equipment used is a sputtering machine used to coat polymers with gold for Scanning Electron Microscopy studies. The structure to be coated is introduced in the chamber filled with nitrogen/argon gas and the coating allowed to take place for several minutes, depending on the thickness desired.

### MANUFACTURING PROCESS

The stents, grafts and distal protection system of this invention are made using braiding technology. A preferred braiding machine is a 24-carrier braiding machine. Prior to braiding the stent, the fibers have to be wound on spindles using a winding machine. The fibers can be wound on the same spindle if there is a necessity for more than one fiber to be on the same spindle or they can be wound onto different spindles. The next step in the process is to transfer the spindles on to the braiding machine, i.e., one spindle on each carrier. The braiding process is then started and the fibers are allowed to intertwine. A mandrel of appropriate diameter and geometry is used to control the shape and size of the produced device.

### HEAT SETTING

In the devices of this invention, memory can be set for a specific configuration using the heat-setting process. To achieve this, the device of this invention is shrunk or compressed together on the mandrel and secured in that configuration using tape at both ends. It is the placed in an oven and heated, such as, for example, to 150° C, in the case of polyester fiber, and then cooled to room temperature slowly. This is called heat-setting which locks-in a specific configuration in the memory of the structure. After removing the mandrel from the oven, the stent is taken off the mandrel and is now ready for use. In the heat-setting process, the intertwining fibers do not necessarily melt and bond together. The heat-setting process can be combined with pressure to introduce ridges-at the intersection of the fibers in the stents, and these ridges can provide additional mechanisms of memory because when the stent is released the fibers become locked at the ridges and regain the heat-set shape.

Heat-setting can also be used to obtain various device configurations. This can be achieved by transferring the device to mandrels of the desired shapes, compressing and heat-setting as described above. Another major advantage of heat-setting is that it can be used to impart different porosities to the devices, depending on the extent of compression employed before heat-setting. The greater the compression, the lower the resulting porosity of the device.

Variations of the stent and graft devices can be used as distal protection systems alone or in combination with another stent device. The function of the distal protection element is to catch any embolic debris such as clots, dislodged plaques and the like, which might be generated at the working area during stent deployment or other endovascular procedures.

### BRIEF DESCRIPTION OF THE DRAWINGS

For a more complete understanding of the invention, reference is hereby made to the drawings, in which:
FIGURE 1 is a schematic perspective view of a tubular prosthesis produced by the braiding process;
FIGURE 2 is a schematic perspective view of the tubular prosthesis showing the intertwining in greater detail;
FIGURE 3 is a greatly enlarged representation of an alternative elastomeric fiber of a prosthesis according to the invention, wrapped with discrete fiber segments;
FIGURE 4 is a greatly enlarged representation of an alternative elastomeric fiber of a prosthesis according to the invention wrapped with continuous fibers;
FIGURES 5a-b are schematic perspective views of a tubular prosthesis in which both heat-settable polymeric and non heat-settable fibers are used (Type 2 structure);
FIGURE 6 provides schematic descriptions of the coated end and a fused end of the stent;
FIGURES 7a-d are a schematic illustration of the heat-set concepts of the present invention;
FIGURE 8 is a schematic of the introduction of ridges by pressure to provide memory to the stent;
FIGURE 9 is a schematic showing of the metal protrusions that may be used for stent anchorage;
FIGURES 10a-d are a schematic description of the process of the stent placement into the catheter and its deployment in a blood vessel;
FIGURES 11a-11c are schematic illustrations of a distal protection system as a variant of the stent of Fig. 1; and
FIGURE 12 is a schematic illustration of one embodiment of the invention shown in Fig. 11.

### DETAILED DESCRIPTION OF THE PREFERRED EMBODIMENT

The elastomeric fiber used in Type 1 structures in accordance with the invention can be natural elastomers, synthetic elastomers, or combination thereof. Natural elastomers include natural rubber. Synthetic elastomers which can be used include, but are not limited to, polyisoprene, polybutadiene and their copolymers, neoprene and nitrile rubbers, polyisobutylene, olefinic fibers such as ethylene-propylene rubbers, ethylene-propylene-diene monomer rubbers, and polyurethane elastomers, silicone rubbers, fluoroelastomers and fluorosilicone rubbers. The preferred elastomeric fibers are polyurethane or silicone elastomers. The fibers used in accordance with the present invention can be mono-filaments or multi-filament fibers and additionally they may be twisted. It should be noted that the foregoing description of the elastomeric fibers is not meant to be limiting. The elastomeric fiber of this invention can be any fiber which has sufficient elastic modulus and extensibility to provide the requisite longitudinal, torsional and radial resiliency. The preferred placement of these fibers is in the axial direction.

The non-elastomeric fibers used as a first component in accordance with Type 2 of the invention can be a metal fiber, a natural fiber, a synthetic fiber or any combination thereof. Examples of metal fibers that can be used include, but are not limited to, stainless steel, inconel, nitinol and titanium. Natural fibers which can be used, but are not limited to, include silk, wool and cellulosic fibers. Synthetic fibers include but are not limited to polyamides such as nylons, polyesters, rayon, polyethylene, polypropylene, polyacrylonitrile, acrylics, polytetrafluoroethylene, polylactic acid, polylactic/glycolic acid, and copolymers, terpolymers and derivatives thereof. The preferred non-elastomeric material is a metal, most preferred being stainless steel, inconel (a nickel/chromium/iron alloy) and nitinol. The fibers in accordance with the present invention may be monofilaments or multi-filiment fibers. Additionally the fibers may be twisted. It should be noted that the foregoing description of the non-elastomeric fibers is not meant to be limiting. The non-elastomeric fiber of this invention can be any fiber which is biocompatible, corrosion resistant, and capable of providing the necessary radial rigidity, transverse elasticity, and resistance to deformation necessary for the tubular prosthesis.

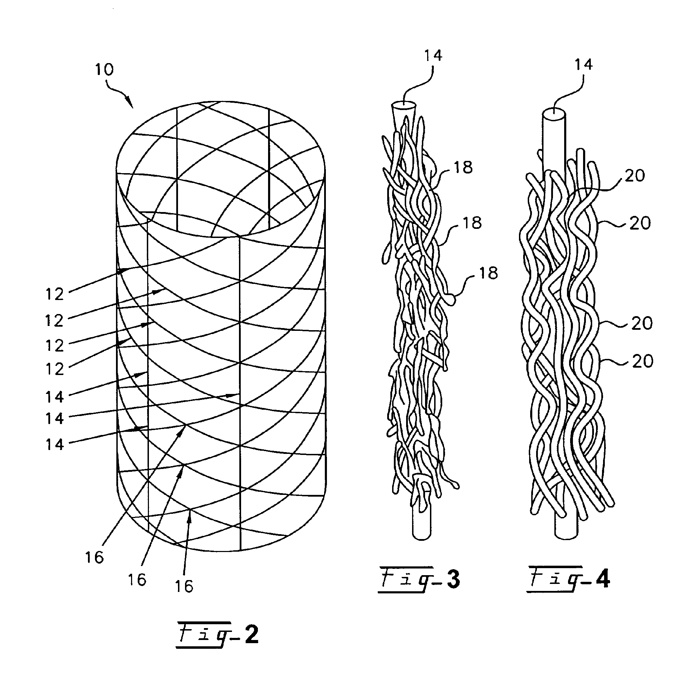

The elastomeric fiber can be wrapped with other fibers. The wrapping can be partial or complete with respect to coverage of the surface of the elastomeric fiber with the wrapping fiber. The elastomeric fibers for this structure can be wrapped with any polymeric biocompatible fiber such as those noted above. Suitable wrapping fibers are, for example, polyesters, cotton, rayon and nylon. The preferred wrapping fiber is a polyester. The wrapping of the elastomeric fiber serves many purposes. One purpose served by the wrapping is that the elastomeric fiber is protected from damage by the other fibers in the structure (used in the braid direction). A second purpose served by wrapping is an effective means of providing the desired surface coverage to the tubular prosthesis. Also, the wrapping of the elastomeric fibers helps to control and stabilize the elastic recovery of the tubular prosthesis and additionally provides extra resistance in the form of friction to help keep the elastomeric fibers properly positioned. Additionally, the wrapping fibers may be treated with therapeutic agents by, growth factors, anti-coagulants, or hormones, for example, dipping the fibers into such agents. After deployment, these agents are released over time in to the body. The extent to which the elastomeric fiber is wrapped can be varied according to the desired end use and desired degree of elasticity of the material, based on the above purposes of the wrapping. Wrapping of the elastomeric fibers can be accomplished by any conventional wrapping process, including simply wrapping by hand, and, preferably, spinning fibers on to the elastomeric fiber. The elastomeric fibers can also be wrapped using continuous fibers or filaments, or discrete fibers, or staple fibers, such as cotton as shown in Figs. 3 and 4. The diameter of the wrapping fibers can be of nano or micro-denier.

The different types of fibers selected for producing the prosthesis are co-braided to produce the desired shape. It should be noted that the tubular prosthesis, in most applications are tubular, but in certain applications they do not necessarily have to be tubular, as is the case of the distal protection system. Further, it is to be understood that tubular includes circular, oval and irregular cross-sections. The prosthesis should be generally tubular and in any event, flexible and elastic enough to conform to the three dimensional shape of the vessel replacement when used as a stent or graft. The outer diameter and the inner diameter of the tubular prosthesis are determined by the intended end use of the prosthesis. Blood vessels in different locations and different patients have different inner and outer diameters, and stents and grafts used to treat and replace such vessels should have corresponding diameters.

The first component and the second component can be intertwined by any method, but are preferably braided in a conventional manner. Methods of braiding fibers and braiding in general have been described in literature; see, e.g., Ko, Frank K, "Braiding", *Manufacturing Processes*, (1987) and Ko, Frank K., "Preform Fiber Architecture for Ceramic-Matrix Composites", *Ceramic Bulletin*, Vol. 68, No. 2, (1989), the entire contents of which are herein incorporated by reference.

The number of different elastomeric and non-elastomeric fibers used will depend upon the desired end use of the material, the overall diameter of the final tubular prosthesis, the specific material used, and the extent to which, if any, the elastomeric fiber is wrapped. For example, if the material is to be used in an application in which the material requires very low porosity, greater numbers of wrapped elastomeric fibers should be used. As such fiber numbers will vary with porosity requirements. Fiber number will also vary with size. A tubular prosthesis with a larger diameter will usually need a larger number of fibers than prosthesis of smaller diameter.

In one embodiment, the ends of the tubular prosthesis are preferably coated to prevent fraying. By coating the ends of the tubular prosthesis and preventing fraying, damage to the blood vessel walls and difficulty in handling can be minimized. Also, by providing the coatings on each end of the tubular prosthesis with some extra texture, anchorage of the prosthesis within the blood vessel can be improved. Suitable coatings may include any biocompatible material. The coating should also preferably be an elastomeric material, such as biocompatable silicones or polyurethanes. The coating can be applied by dipping the end of the tubular prosthesis into a curable silicone or polyurethane coating composition, and subsequently curing the coating. Heat or ultra-violet light can be applied in some cases to enhance the cure rate. Brushing or spraying of the coatings can also accomplish coatings of the ends of the prosthesis. Curing of the coatings can be accomplished by any conventional method, however, self-curing and ambient-curable coatings are preferred as heat can damage the elasticity of the elastomeric materials in the prosthesis. Additionally, self-curing and ambient-curable coatings shrink less upon cure and are also preferable for that reason. Generally, the coating should be applied to the extent necessary to reduce fraying, which in most cases is about four picks or intersections of the intertwined fibers. This can be accomplished by coating from about 1 mm to about five mm as measured longitudinally on each end of the tubular prosthesis. However, the coating may extend further if desired and, indeed, if the intended application permits, can cover the entire prosthesis. Generally, tubular prostheses, which require less porosity, can permit use of more extensive coatings, and tubular prostheses, which may require substantial cutting of length, may also have more extensive end coatings. The thickness of the coating can be about from 1 micron to about 500 microns, and is preferably from about 20 microns to about 200 microns.

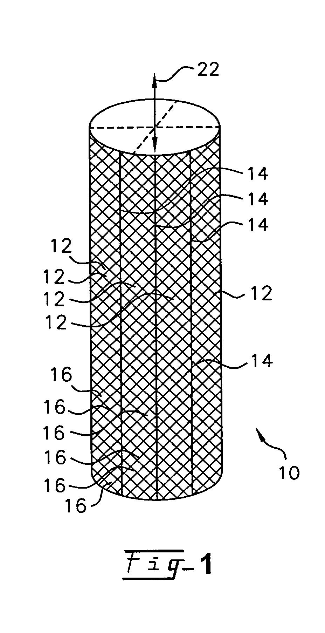

In the drawings, like numerals are used to indicate like elements throughout Fig. 1 shows a tubular prosthesis 10, generally, for use as vascular stent or graft in accordance with the invention. The heat-settable and non-elastomeric fibers 12 are disposed in an helical configuration, wherein oppositely wound helical fibers are intertwined over and under each other to form multiple intersections 16 of non-elastomeric fibers 12 and referred to herein as braid yarns. The elastomeric fibers 14 are disposed longitudinally on the device and generally parallel with respect to the center axis 22 of the tubular prosthesis 10, and referred to herein as axial yarns, and are interwoven with the braid yarns 12.

Fig. 2 shows a magnified view of the relationship between the braid yarns 12 and the axial yarns 14. The axial yarns 14 are intersected in such a fashion that they pass over and under successive braid yarns 12. The positioning of the axial yarns 14 with respect to the intersection 16 of oppositely wound helices can be varied. The positioning shown in Fig. 2 is not meant to be limiting and specifically, it should be noted that it is not required that the axial yarns 14 be, in some manner, interwoven over and under successive braid yarns 12.

Fig. 3 shows a greatly enlarged representation of an elastomeric fiber 14 wrapped with discrete fiber segments 18.

Fig. 4 shows a greatly enlarged representation of an elastomeric fiber 14 wrapped with continuous fibers 20.

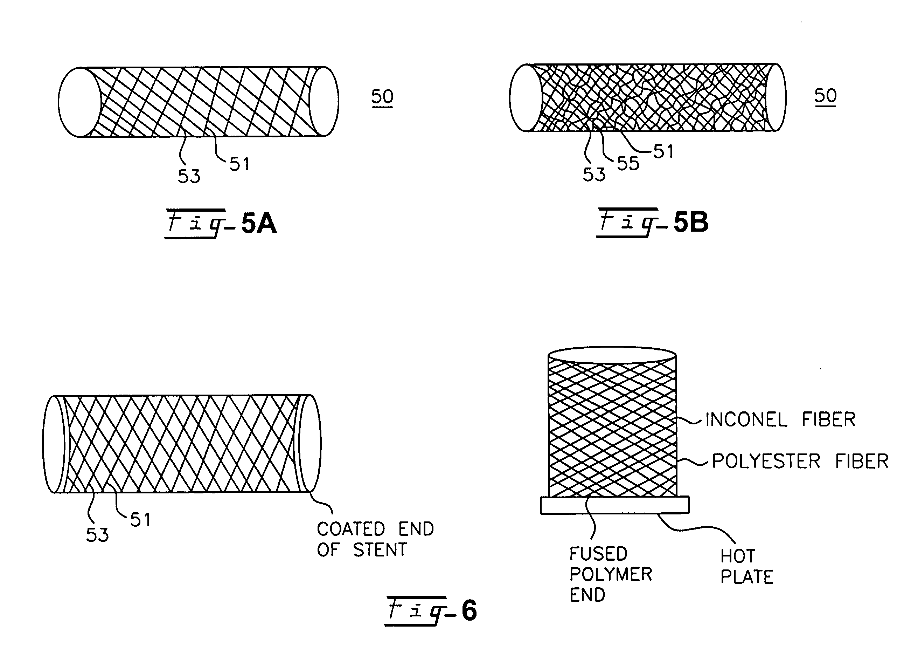

Fig. 5a shows a preferred structure 50 in accordance with the invention where both a metal fiber 51, inconel, and a polyester mono-filament fiber 53 are used, whereas in Fig. 5b, a polyester multi-filament fiber 55 is also used.

The primary constituent of the devices described herein is the heat-settable non-elastomeric fiber (mono-filament or multi-filament) which provides the shape, coverage and radial stiffness to the stent of this invention. The preferred fibers are polyester or polyethylene terephthalate as monofilaments. The incorporation of non-elastomeric metal fibers is primarily for the purpose of radio opacity and in certain cases does provide the additional feature of helping in the stent anchorage. The preferred non-elastomeric fibers in Type 2 structures are metallic fibers. Non-metallic, non heat-settable polymeric fibers may also be used in Type 2 structures in conjunction with heat-settable fibers as described above in order to tailor the physical and mechanical properties of the stents. In such cases, these fibers can be any of the single or multi-filament elastomeric or non-elastomeric fibers described above. Fig. 5 shows a stent with a combination of heat-settable and non heat-settable fibers. The preferred metal in this example is inconel, though other radio opaque metal fibers like nitanol and stainless steel can also be used. There is no limitation absolutely to the choice of materials as long as it conforms to the specific requirements of the stent. Further, the devices can be made to have very high surface coverage or with an open porous structure.

Elastomeric fibers/yarns may be used in the axial direction of the braid to provide the required longitudinal flexibility and elasticity and the use of stiff and/or elastic fibers/yarns in the helical direction for the required stiffness. The present invention has successfully produced stents using such a combination of fibers/yarns. Specifically, two-dimensional circular braiding has been used with Spandex yarns in the axial direction and stainless steel filament in the braid direction. However the process can be achieved by many textile processes, including but not limited to braiding, weaving, knitting and/or combinations of the same, while the axial fibers/yarns can be any elastomeric fibers/yarns such as urethanes, silicones and variations of the same, and the braiding fibers can be any polymeric or metallic fiber with the desired size and stiffness such as nitinol, stainless steel, polyester, polytetraflouroethylene, high molecular weight gelspun polyethylene and variations of the same. The braiding fibers can also include the elastomeric fibers described above for additional elasticity in the hoop direction, if required by the specific application. The fiber/yarn combinations mentioned above may be used with or without wrapping. The design of this invention also permits the use of fibers and yarns that are readily imageable during deployment simply replacing one of the axial or braided fibers with an imageable fiber (available in the literature) or simply coating one of the fibers to make it imageable. Another variable in the novel design described herein is the porosity and fiber coverage through all of the stent. This is highly tailorable for a given application through the amount and type of fibers used as well as the particular textile processing technique used.

Stents with high coverage can be made with 9 mono-filament fibers, 9 multi-filament fibers of the same material and 6 metal fibers, each mounted on separate carriers and then braided into a stent. The stent can be braided at a low angle to have a high surface coverage or can be brained at a high angle, then reduced in length on the mandrel and heat-set. Wrapping significantly enhances surface coverage.

Stents with very high coverage can be made with 18 mono-filament and 18 multi-filament fibers, one of each type wound on the same spindle and braided along with six metal fibers.

Stents requiring low thickness can be made with 12 mono-filament fibers, 6 multi-filament fibers and 6 metal fibers as above.

Another example is 12 mono-filament fibers, 6 of them co-wound with 6 multi-filament fibers only and 6 metal fibers.

For insertion into very low diameter catheters, a stent with minimal thickness when shrunk to its lowest diameter is desired.

Stents made with only 18 mono-filament fibers and 6 metal fibers each type on separate spindles or one metal and one mono-filament co-braided onto the same spindle are also contemplated. Stents can have their ends coated or fused together by heat, as in Fig. 6. Heat setting of the stent enables the stent structure to remain intact without fraying.

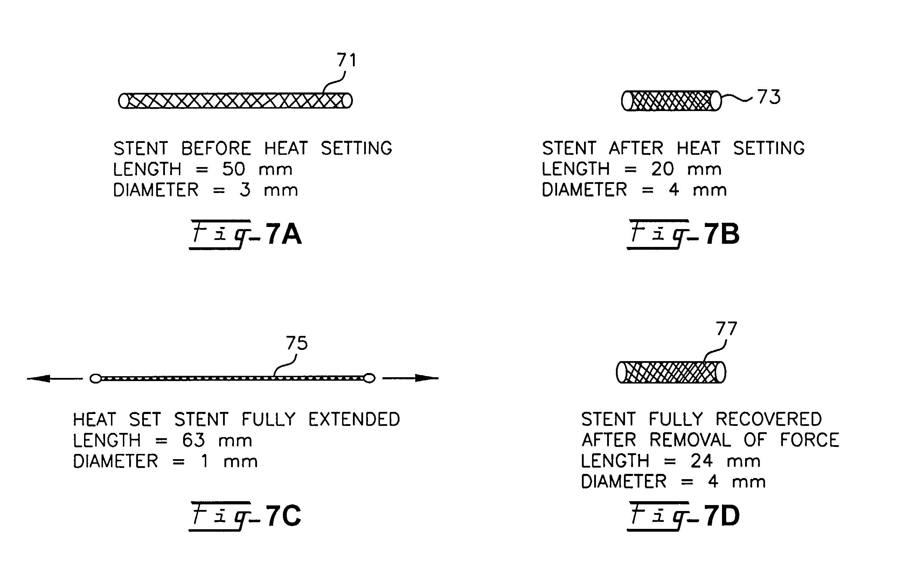

Figs. 7a-d represent the preferred embodiment of the present invention, where a braided stent 71, is formed in either type 1 or type 2 form, as described above, so that the stent has a predetermined length. All that is required is that the stent 71 have at least some fiber which is capable of holding a heat-set when applied as described herein. Typical dimensions might be a length of 50 mm and a diameter of 3 mm. Preferred examples of heat-set capable fibers have been listed above. In Fig. 7b, the stent 73 has been compressed axially to a shorter length and subjected to heat treatment to heat-set stent 73 in this embodiment, so that the length, for example only, might be 20 mm and the diameter 4mm, since the same amount of fiber is present. This heat-set version of the stent 73 may be coated, as described below, or used as is. When stent 73 is used, it is extended as shown in Fig. 7c as stent 75, having an extended length of, for example, 63 mm, which results in a small diameter of 1 mm, for insertion into a body vessel as intended. When the force of extending the stent 75 is released after placement in the body, the stent 77 recovers to a useful size, such as, for example, one with a length of 24 mm and a diameter of 4mm.

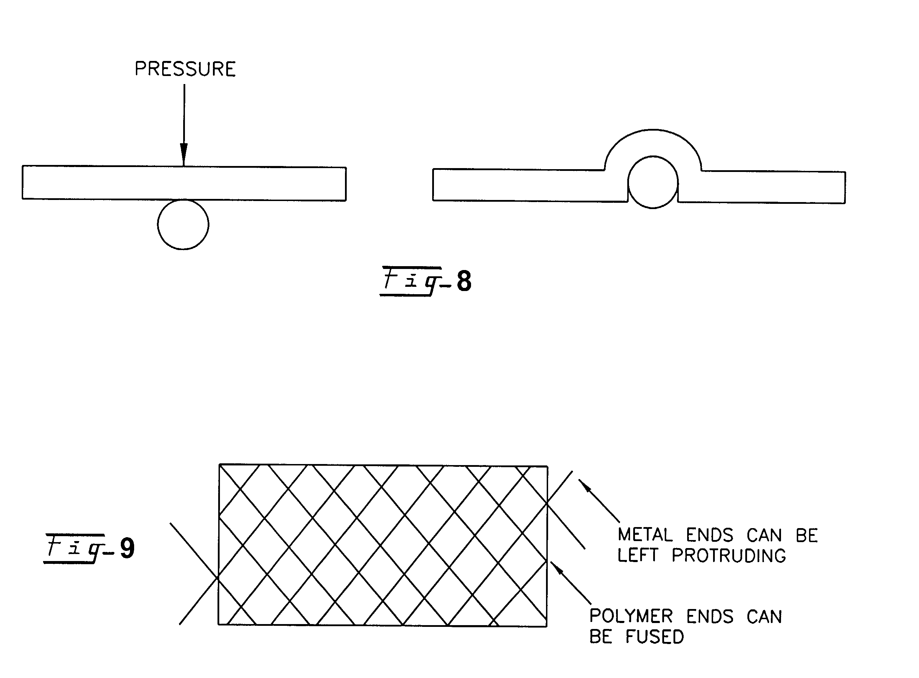

As described earlier, the heat-setting can be combined with pressure to provide more memory, as in Fig. 8.

Stent anchorage can be accomplished in at least two ways. One is to braid a stent to the exact diameter as the artery and then let the metal protrusions from its end dig into the artery and anchor the stent, as in Fig. 9. A second way is to braid a stent having a slightly larger diameter than the artery into which it is going to be placed.

The radial pressure on the stent and frictional force will help anchor the stent at its specific site. In this particular case, after the stent end is fused the metal protrusions from the ends can be trimmed off or can be left there for additional anchorage.

A variety of stent types conforming to Type 1 and Type 2 can be made by varying the braiding angle to have an increased or reduced coverage. Variety of filament diameters ranging from 250 to 60 microns can be used for the mono-filament fibers. Stent diameter can be adjusted by selecting the appropriate diameter of the mandrel onto which the fibers are braided.

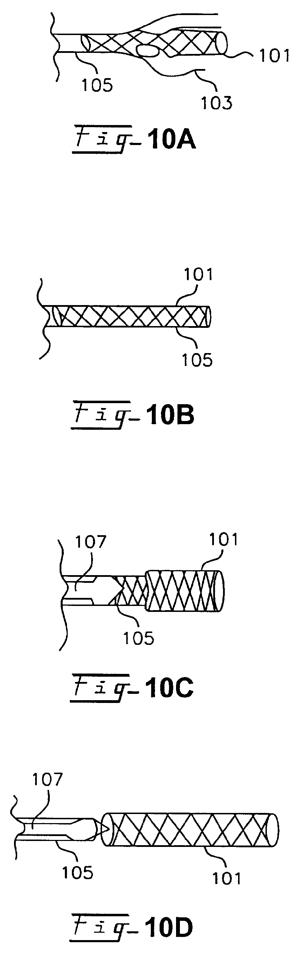

Figs. 10a-d are schematic drawings showing the preferred process of this invention as presently contemplated, for stent placement into a catheter and its deployment. The stent 101 is squeezed by hand 103 and manually inserted into the catheter 105 as shown in Fig. 10a. Once the stent is lodged completely inside the catheter 105 as in Fig. 10b, the catheter is introduced into the body using standard techniques used currently by surgeons in stent deployment and pushed to the location where it needs to be deployed or placed. At the desired location the stent is pushed out of the catheter using another catheter, shown in Figs. 10c and 10d, typically a balloon catheter 107 in the deflated state.

The novel design of the present invention involves the use of a variety of small diameter fibers to provide the required tubular structure with good radial stiffness and extensibility, optionally braided with metal fibers to render it radio opaque. The incorporation of multi-filament fibers is subject to the needs of the stent application and requirements.

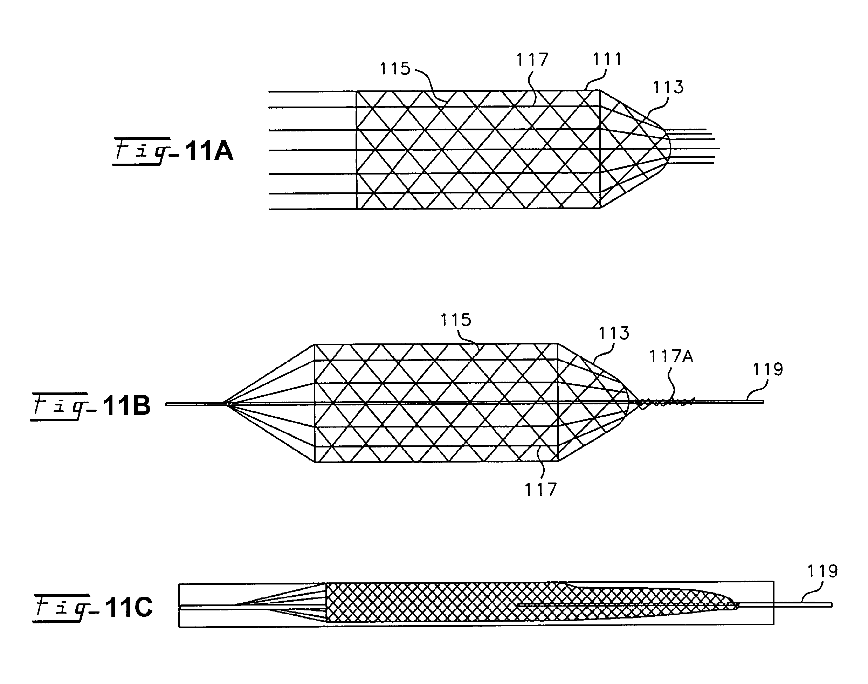

Another variation of the present invention is to use the stent as described herein as a distal protection system. The stent and the distal protection component may be present in one system or comprised of two components. The stent and the distal protection device can be deployed separately at the discretion of the physician or surgeon. Fig. 11a illustrates one form 111 of this distal protection device embodiment, where it is shaped like a net or umbrella shape, such that the leading end 113 of the device 111 is placed in front of the stent before the device is inserted into the body. The function of the distal protection element is to catch any embolic debris, such as clots, dislodged plaques and the like, which might be generated at the working area during stent deployment or any endovascular procedure. The debris then can be removed from the vascular system by using the distal protection device 111, by closing the distal end 113 as shown in Fig. 11b. The essence of the distal protection element is to prevent debris from being flushed into the coronary, limb, or brain arteries, thus inducing an ischemia to the end organ or/and a stroke.

While placed in the artery, the distal protection element has to maintain blood flow, as in Fig. 11a, to the distal organ such as the brain, heart, extremity or any supplied organ, while also being properly placed within the artery. By appropriate manipulation, the distal protection element is closed, as in Fig. 11b, to entrap any debris for removal. It is contemplated that the distal protection element will be fixedly attached to the stent portion when the stent itself is to be removed, as in some procedures. When the stent is to remain in the blood vessel or the like, the distal protection element can either be completely detached or detached in-situ and removed with the debris while leaving the stent in place. As shown in Figs. 11a and 11b, the distal protection element is woven as described for the stent design with heat-settable polymer filaments and/or hybrid compositions of such polymers with metal filaments in the braid direction and axial filaments, which are preferably metal but also can be non-elastomeric polymeric filaments. It is also contemplated that this element may be heated to set it in a useful size, reduced in diameter by extension for insertion, and released to the original size as desired.

More particularly, the filaments 115 in the braid direction of distal protection element 111 in Fig. 11a are, at least in part, heat-settable, though hybrid forms including metal filaments may also be used in the braid direction. The axial filaments 117 are metal or polymeric, as desired. A guide wire 119 is used, and the axial filaments 117 are used to fix the stent 111 to the wire 119, but axial fibers are optional here because the braid direction fibers can also be used for this purpose. Note that the shape is more "cigar-shaped" than in other embodiments, such as Figs7a-d. Distal protection element 111 is fixed on guide wire 119 and any protruding axial filaments 117a, at point B in Fig. 11b, are attached to the guide wire 119, firmly, by any conventional joining methods (i.e., cutting a notch and tying the filaments at the notch, using a ring-like structure to which all filaments are tied, welded or glued, or by direct welding, soldering or gluing. Point A is selected such that the stent body is close to the fully deployed configuration, or unstretched free condition.

The entire assembly is then introduced into a catheter delivery system, using procedures previously described. In the shape shown in Fig. 11c, the distal protection system 111 is in a crimped condition with a minimal profile. The distal protection is then deployed by pushing it out as previously described by a balloon catheter (not shown in this Figure) or otherwise.

Wire 119 serves as a guide wire for most stent systems and for angioplasty balloon catheters and other simple catheters that may be placed in front of the distal protection device 111. When deployed, device 111 takes the configuration shown in Fig. 11b, and pushing a catheter system forward will retrieve it. This will have the effect of closing the front end because the filaments are attached at point A, and then the device 111 is pulled in and retrieved. Alternatively it is possible to design the stent such that it closes the front end of device 111 after the debris is captured in it by twisting guide wire 119 to apply a torque. Once the closed end is completed, it can then be withdrawn by pulling guide wire 119. Of course, to be completely effective, device 111 should be braided to have a pore size that is smaller (less than about 10 microns) than the embolic debris expected to be entrapped.

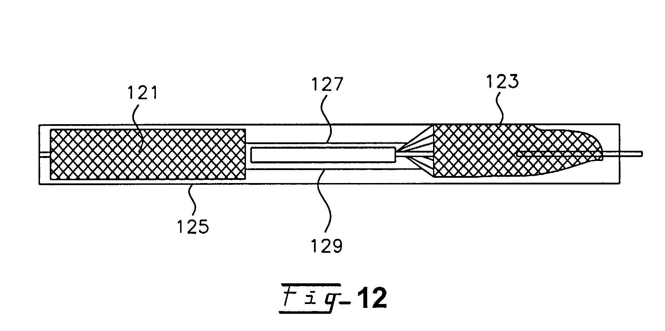

The device shown in Figs. 11a-c may be used as a separate unit ahead of the stent on the delivery system, or as a single piece as shown in Fig. 12. Here the stent 121 and distal protection device 123 are contained in catheter 125 and may, optionally be spaced by spacer 127 and/or connected by connector 129, both of which can be broken in-situ.

As another embodiment, a thin coating can be applied to the stent of this invention to alter its surface properties. Such coatings can be applied on either or both of the stent surfaces depending on the desired effect. The nature of the coating material can vary depending on the properties desired. If the coating material is such that it swells when exposed to body fluids, the net result is a reduction of the stent porosity. This may be desirable when the stent is used for the treatment of aneurysms to minimize seepage of blood to the aneurysm itself. Coating materials useful for such purpose can be natural or synthetic and can be chosen to provide the desired extent of swelling when exposed to body fluids. Examples are cellulosic materials, collagen, polyvinyl alcohol and its copolymers, polyvinyl alcohol/polyvinyl pyrolidone combinations, polyacrylic acid and its modifications, poly(isobutylene- maleic anhydride) copolymers and their modifications, and other such materials as desired. The device of this invention may be used as a delivery system or platform for drugs, hormones, genes, growth factors and the like.

It will be appreciated by those skilled in the art that changes could be made to the embodiments described above without departing from the broad inventive concept thereof. While particular embodiments of the present invention have been illustrated and described, it is not intended to limit the invention to any specific embodiment. The description of the invention is not intended to limit the invention, except as defined by the following claims.

## Claims

1. A stent device for insertion into a body as a stent or graft to a predetermined position in a body location, comprising:
a braided stent having heat-settable polymer filaments, said stent being compressed or configured to a desired shape and heat-set in said shape.

2. The device of claim 1, wherein:
the heat-settable polymer filaments are selected from poly(ethlyene terephthalate), polyethylene or a biodegradable polymer selected from polylactic acid or poly(lactic-glycolic) acid; said device further including non heat-settable filaments; and
wherein said stent is axially extended and inserted into a delivery system device for introduction into a predetermined body location, whereby said stent returns to substantially its heat-set predetermined size and shape upon release from said delivery system device.

3. A debris protection device for use with a stent device for insertion into a body as a stent or graft to a predetermined position in a body location, comprising:
a braided component made from heat-settable polymer filaments and including a distal end and a proximal end, said proximal end including attachment means for attachment of said component to a stent device;
said distal end of said component including closure means for substantially closing said distal end and mounted on said distal end to entrap body debris at said body location; and
said closure means comprising an insertion guide wire fixedly mounted to said distal end for sequentially inserting, guiding, closing and removing said component from said body location.

4. The device of claims 1, 2 or 3, wherein said stent includes a natural, synthetic or metallic coating on at least one surface of said stent.

5. The device of claim 4, wherein said coating comprises a coating material adapted to swell upon exposure to body fluids to reduce the porosity of said stent.

6. The device of claim 4, wherein said coating material is selected from the group of cellulosic materials, collagen, polyvinyl alcohol and its copolymers, polyvinyl alcohol/polyvinyl pyrolidone combinations, polyacrylic acid and its modifications, poly(isobutylenemaleic anhydride) copolymers and their modifications.

7. The device of claims 1 or 4, wherein heat-settable polymer or said coating contains a therapeutic drug, hormone, growth factor or gene.

8. A method of forming a stent device for insertion into a body as a stent or graft to a predetermined position in a body location, comprising the steps of:
braiding a stent having heat-settable polymer filaments;
compressing said braided stent axially to a shorter length; and
heat-setting said stent in a predetermined shortened size.

9. The method of claim 8, which further includes the steps of:
adding non heat-settable filaments in the braiding process; and
selecting the heat-settable polymer filaments from poly(ethlyene terephthalate), polyethylene or a biodegradable polymer selected from polylactic acid or poly(lactic-glycolic) acid; and
axially extending said stent and inserting said extended stent into a delivery system device for introduction into a predetermined body location, whereby said stent returns to substantially its heat-set predetermined size and shape upon release from said delivery system device.

10. The method of claim 8, which further includes the step of providing a debris protection component including a distal end and a proximal end, said component being attached to the distal end of said stent device, wherein said debris protection component is provided with an insertion guide wire and said distal end is fixedly mounted to said guide wire for closure of said distal end upon removal of said debris protection component to entrap body debris at said body location.

11. The method of claim 8, which further includes the step of providing a natural or synthetic coating on at least one surface of said stent.

12. The method of claim 8, wherein said coating comprises a coating material adapted to swell upon exposure to body fluids to reduce the porosity of said stent.

13. The method of claim 11, wherein said coating material is selected from the group of cellulosic materials, callogen, polyvinyl alcohol and its copolymers, polyvinyl alcohol/polyvinyl pyrolidone combinations, polyacrylic acid and its modifications, poly(isobutylenemaleic anhydride) copolymers and their modifications.

14. The method of claims 8 or 11, wherein heat-settable polymer or said coating contains a therapeutic drug, hormone, growth factor or gene.
